Europäisches Patentamt

European Patent Office

Office européen des brevets

(19)

(11) Veröffentlichungsnummer : **0 407 840 B1**

# (12) EUROPÄISCHE PATENTSCHRIFT

(45) Veröffentlichungstag der Patentschrift :
**10.11.93 Patentblatt 93/45**

(51) Int. Cl.$^5$ : **C07C 11/02, C07C 1/20**

(21) Anmeldenummer : **90112520.3**

(22) Anmeldetag : **30.06.90**

(54) **Verfahren zur Herstellung von reinen C4-C7-tert.-Alkenen.**

(30) Priorität : **14.07.89 DE 3923291**

(43) Veröffentlichungstag der Anmeldung :
**16.01.91 Patentblatt 91/03**

(45) Bekanntmachung des Hinweises auf die
Patenterteilung :
**10.11.93 Patentblatt 93/45**

(84) Benannte Vertragsstaaten :
**BE DE FR GB IT NL**

(56) Entgegenhaltungen :
**EP-A- 0 302 336**

(73) Patentinhaber : **EC ERDÖLCHEMIE GMBH
Alte Strasse 201
D-50769 Köln (DE)**

(72) Erfinder : **Schleppinghoff, Bernhard, Dr.
Adolf-Kolping-Strasse 5
D-4047 Dormagen (DE)**
Erfinder : **Stüwe, Arnd, Dr.
Berta-von-Suttner-Strasse 34
D-5090 Leverkusen 1 (DE)**
Erfinder : **Niederberger, Hans-Ludwig, Dr.
Goethestrasse 63
D-4047 Dormagen (DE)**
Erfinder : **Scheef, Hans-Volker
Goethestrasse 69
D-4047 Dormagen (DE)**
Erfinder : **Grub, Joachim, Dr.
Ostpreussenallee 10
D-4047 Dormagen (DE)**

(74) Vertreter : **Müller, Gerhard, Dr. et al
BAYER AG Konzernverwaltung RP Patente
Konzern
D-51368 Leverkusen (DE)**

EP 0 407 840 B1

Jouve, 18, rue Saint-Denis, 75001 PARIS

## Beschreibung

Die vorliegende Erfindung betrifft die Herstellung von reinen $C_4$-$C_7$-tert.-Alkenen durch Spaltung ihrer Ether von 2- und/oder 3-wertigen Alkoholen.

Tertiäre Alkene sind wichtige Vorprodukte für Oligomere, beispielsweise für Lösungs- und Schmiermittel, für Polymere und Copolymere und für höherwertige Chemikalien, wie Pinacolin, Neocarbonsäuren, Isopren und anderen. Solche tertiären Alkene fallen in roher Form beispielsweise bei der thermischen oder katalytischen Crackung von Leichtbenzin, Naphtha und anderen geeigneten Ausgangsmaterialien oder bei deren Dehydrierung und/oder Isomerisierung an; sie liegen hierbei meist im Gemisch mit einer Vielzahl gesättigter und ungesättigter Begleitstoffe vor, deren destillative Trennung schwierig und kostspielig ist, da man von Destillationsschnitten ausgeht, in denen ähnlich siedende Gemischbestandteile gleicher oder benachbarter Anzahl an C-Atomen vorliegen.

Die Isolierung der tertiären Alkene erfolgt daher über die selektive Umsetzung, Abtrennung des Umsetzungsproduktes und Zersetzung der abgetrennten reinen Umsetzungsprodukte. So können die bei einer solchen Umsetzung mit Alkanolen gebildeten tert.-Alkyl-alkylether von den Begleitstoffen der tert.-Alkene abgetrennt und rein erhalten werden. Die hierzu bekannten Methoden der Destillation, Azeotropdestillation, Extraktivdestillation und andere sind jedoch häufig aufwendig und, wenn die Siedepunktslage des Ausgangskohlenwasserstoffgemischs und des abzutrennenden Ethers näher beieinanderliegen, sehr aufwendig.

Die in der beschriebenen Weise rein erhaltenen tert.-Alkyl-ether können dann wieder in die zugrundeliegenden reinen tertiären Alkene und die Alkanole gespalten werden (siehe z.B. DE-A-3210435 und EP-A-302336). Bei dieser Spaltung ist es jedoch nicht ganz vermeidbar, unerwünschte Nebenprodukte zu erhalten, insbesondere die Dialkylether aus den bei der Spaltung entstehenden Alkanolen. Hierdurch werden einerseits die Alkanole, die üblicherweise in die Veretherung recyclisiert werden, in Form dieser Dialkylether aus dem gesamten System herausgenommen, andererseits wird durch die Bildung dieser Dialkylether die Aufarbeitung des zu den reinen tert.-Alkenen führenden Reaktionsgemisches komplizierter. Eine noch weitere Nebenproduktbildung ist die Bildung von tert.-Alkanolen aus den gewünschten tert.-Alkenen und dem bei der Etherbildung anfallenden Wasser.

Es wurde nun gefunden, daß die genannten Schwierigkeiten umgangen werden können, wenn man zur Herstellung von reinen $C_4$-$C_7$-tert.-Alkenen von deren Ethern mit 2- und/oder 3-wertigen Alkoholen ausgeht.

Es wurde ein Verfahren zur Herstellung von reinen $C_4$-$C_7$-tert.-Alkenen durch Spaltung ihrer Ether an sauren Katalysatoren bei erhöhter Temperatur gefunden, das dadurch gekennzeichnet ist, daß man die $C_4$-$C_7$-tert.-Ether von 2- und/oder 3-wertigen Alkoholen einsetzt und bei 60-250°C spaltet.

Als $C_4$-$C_7$-tert.-Alkene, aus denen die zu spaltenden Ether aufgebaut sind, seien beispielsweise genannt: i-Buten, 2-Methyl-buten-1, 2-Methyl-buten-2, die verschiedenen isomeren Hexene und Heptene, bei denen sich die Doppelbindung an einem tertiären C-Atom befindet, und weiterhin solche isomeren $C_5$-$C_7$-Alkene, bei denen eine Doppelbindung, die zunächst nicht am tertiären C-Atom steht, durch Isomerisierung unter den Reaktionsbedingungen an das tertiäre C-Atom wandert. In bevorzugter Weise handelt es sich bei solchen tert-Alkenen um i-Buten, 2-Methyl-buten-1 oder 2-Methyl-buten-2. Für die Veretherung können solche tert.-Alkene einzeln oder im Gemisch mehrerer von ihnen vorliegen. Zur Veretherung liegen solche tert.-Alkene im allgemeinen im Gemisch mit anderen gesättigten oder ungesättigten Kohlenwasserstoffen vor, wobei in bevorzugter Weise auch von Destillationsschnitten ausgegangen wird, in denen die miteinander vergesellschafteten tert.-Alkene, andere Alkene und gesättigte Kohlenwasserstoffe im wesentlichen die gleiche Anzahl von C-Atomen haben. 2- oder 3-wertige Alkohole, die den zu spaltenden Ethern zugrundeliegen, sind beispielsweise Ethylenglykol, Propandiol-1,2, Propandiol-1,3, Butandiol-1,2, Butandiol-1,3, Butandiol-1,4, Butandiol-2,3, entsprechend aufgebaute Pentan- und Hexandiole, weiterhin die Ether solcher Diole untereinander, wie Diethylenglykol, Triethylenglykol, Dipropylenglykol, Tripropylenglykol und andere, Glycerin, Trimethylolpropan und Formosen mit einer niedrigen OH-Funktionalität, wie sie durch Selbstkondensation von Formaldehyd zur Formose und anschließender spaltender Reduktion an Ni-Katalysatoren zum Formit hergestellt werden können, der im wesentlichen 2-wertige und 3-wertige Alkanole enthält (DE-OS 27 56 270).

In bevorzugter Weise werden zur Spaltung die $C_4$-$C_7$-tert.-Ether von 2-wertigen Alkoholen und ihren Oligoethern eingesetzt, die an beiden Enden jeweils noch eine freie OH-Gruppe tragen. In besonders bevorzugter Weise werden die Ether von Ethylenglykol, Propandiol-1,2 oder deren Diethern eingesetzt.

Als Katalysatoren für die Etherspaltung können saure anorganische oder organische Stoffe eingesetzt werden, beispielsweise saures $SiO_2$ oder $Al_2O_3$ mit großer Oberfläche, Silico-Aluminate, Zeolithe, saure Oxide anderer Elemente, Phosphorsäure, Schwefelsäure oder sauer reagierende Salze. Von diesen sind die festen unlöslichen Stoffe bevorzugt. Bei den organischen Spaltkatalysatoren kann es sich ebenfalls um stark saure Substanzen, wie Sulfonsäuren oder halogenierte Carbonsäuren handeln, bevorzugt jedoch um unlösliche polymere Kationenaustauscher in der $H^+$-Form. Solche Kationenaustauscher werden beispielsweise durch Um-

2

setzung von olefinisch ungesättigten Monomeren, wie Styrol oder Acrylsäurederivate mit Vernetzern, wie Divinylbenzol, erhalten. Weitere Matrices für Ionenaustauscher sind beispielsweise Phenol-Formaldehyd-Harze, Polyalkylsiloxane und andere dem Fachmann bekannte Die kationenaustauschende Gruppe ist beispielsweise die phenolische OH-Gruppe, die Carboxylgruppe, wie sie beispielsweise aus der Verseifung von Acrylsäurederivaten entsteht oder die durch nachträgliche Sulfonierung an den aromatischen Kernen angebrachte Sulfonsäuregruppe. In bevorzugter Weise werden Kationenaustauscher eingesetzt, die aus Sulfonsäuregruppen enthaltenden Styrol-Divinylbenzol-Harzen in der $H^+$-Form oder aus Sulfonsäuregruppen enthaltenden Polyalkylsiloxanen bestehen Solche Katalysatoren, deren Vorkommen und Herstellung, sind dem Fachmann bekannt.

Das beanspruchte Verfahren kann bei einer Temperatur von 60-250°C, bevorzugt 60-150°C durchgeführt werden. In den oberen Teilen dieses Temperaturbereichs wird im allgemeinen nur bei Anwendung anorganischer Spaltkatalysatoren, beispielsweise bei Anwendung von sauren Zeolithen, gearbeitet. Eine bevorzugte Form ist die Benutzung von Kationenaustauschern in der $H^+$-Form bei einer Temperatur von 60-130°C, bevorzugt 80-120°C. Sulfonsäuregruppen enthaltende Polyalkylsiloxane werden bevorzugt bei 60 - 160°C eingesetzt.

Als Belastung für den Spaltkatalysator kommt eine LHSV (Liquid Hourly Space Velocity) von 0,25 bis 20 Liter Ether oder Ethergemisch pro Liter trockenem Spaltkatalysator pro Stunde in Frage.

Die Etherspaltung kann in flüssiger oder gasförmiger Phase und im wesentlichen drucklos vorgenommen werden.

Bei der Spaltung entsteht neben dem tertiären Alken der zugrundeliegende 2- und/oder 3-wertige Alkohol. Das tertiäre Alken und der genannte Alkohol werden entweder sofort während der Spaltreaktion oder in einer der Spaltreaktion nachgeschalteten Destillationskolonne getrennt.

Der mehrwertige Alkohol kann in einer bevorzugten Verfahrensvariante in eine Veretherungsstufe zurückgeführt werden, in welcher der zu spaltende Ether gebildet wird. Auf diese Art stellt der im Kreislauf gefahrene mehrwertige Alkohol lediglich das Beförderungsmittel für das aus einem Kohlenwasserstoffgemisch selektiv zu gewinnende tertiäre Alken dar. Es wurde weiterhin gefunden, daß eine solche Veretherung dann in einer homogenen Phase abläuft, wenn man dem einzusetzenden 2- oder 3-wertigen Alkohol und dem das tertiäre Alken enthaltenden Ausgangskohlenwasserstoffgemisch Ether der entstehenden Art vor Eintritt in den Veretherungsreaktor zusetzt. In einem solchen Fall wird nicht der gesamte entstehende Ether durch Spaltung zum reinen tertiären Alken umgesetzt, sondern zum Teil gemeinsam mit dem bei der Spaltung entstehenden 2- oder 3-wertigen Alkanol in die Veretherungsstufe zurückgeführt.

Diese bevorzugte Variante der Herstellung von reinen $C_4$-$C_7$-tert.-Alkenen besteht also darin, daß man einen Ether einsetzt, der durch Umsetzung eines ein $C_4$-$C_7$-tert.-Alken enthaltenden Kohlenwasserstoffstroms mit einem 2- und/oder 3-wertigen Alkohol an einem Sulfonsäuregruppen enthaltenden Styrol-Divinylbenzol-Polymer in der $H^+$-Form in Gegenwart von 10 bis 40 Gew.-% des zu spaltenden Ethers bei 20-90°C erhalten wird, wobei die Spalttemperatur 20-120°C über der Veretherungstemperatur liegt.

In besonders bevorzugter Form wird dabei der zuzusetzende Ether im Gemisch mit dem bei der Spaltung zurückgewonnenen 2- und/oder 3-wertigen Alkohol gemeinsam zur Veretherungsstufe geführt.

In besonders bevorzugter Form wird hierbei ein Propandiol-1,2-mono-und/oder -di-tert.-$C_4$-$C_7$-alkylether gespalten und das bei der Spaltung erhaltene Propandiol-1,2 gemeinsam mit nicht gespaltenem Mono- und/oder Diether oder mit nicht vollständig gespaltenem Diether in die Umsetzung zur Veretherung zurückgeführt. Bei einer solchen Veretherung werden 0,4-4 Mol $C_4$-$C_7$-tert.-Alken pro Äquivalent OH-Gruppe eingesetzt.

Als Belastung des Veretherungskatalysators kommt eine LHSV von 0,1 bis 10 Liter gesamtes einzusetzendes zu veretherndes Gemisch pro Liter trockenem Veretherungskatalysator pro Stunde in Frage.

Die Veretherung wird bei Normaldruck oder bei einem solchen erhöhten Druck ausgeführt, daß alle Bestandteile im zu veretherndem Gemisch in der flüssigen Phase vorliegen. Dies ist beispielsweise ein Druck von 1-30 bar, bevorzugt 1-15 bar, besonders bevorzugt der Eigendruck des Systems.

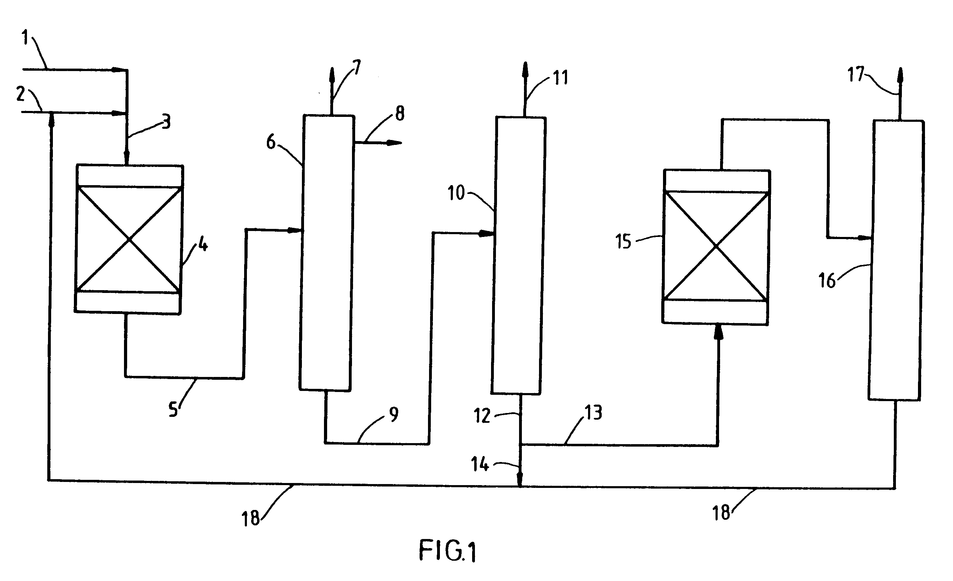

Anhand von Fig. 1 sei das erfindungsgemäße Verfahren der Etherspaltung in Kombination mit der Etherbildung wie folgt dargestellt:

Ein das tert.-Alken enthaltender Kohlenwasserstoffstrom (1), beispielsweise ein sogenanntes Raffinat I im Falle von i-Buten, und ein über die Leitung (18) zurücklaufender 2- und/oder 3-wertiger Alkohol aus der Etherspaltung werden gemeinsam über die Leitung (3) in den Veretherungsreaktor (4) eingespeist. Über die Leitung (2) kann etwa durch Leckagen verlorener 2-und/oder 3-wertiger Alkohol nachgesetzt werden. Das den Veretherungsreaktor verlassende Reaktionsgemisch wird über die Leitung (5) in eine Destillationskolonne (6) geleitet, in der das vom tert.-Alken befreite Kohlenwasserstoffgemisch (im Falle von i-Buten das sogenannte Raffinat II) über Kopf als (7) abgenommen wird. Der oder die Ether sowie etwa überschüssiger mehrwertiger Alkohol werden als Sumpfprodukt über (9) abgenommen. Die Kolonne (6) besitzt unterhalb des Kolonnenkopfes noch eine Abnahme (8), über die etwa durch das mehrwertige Alkanol eingeschlepptes Wasser oder daraus mit dem tert.-Alken gebildetes tertiäres Alkanol abgezogen werden. Sollten sich niedere Oligomere des tert.-Alkens gebildet haben, können diese ebenfalls über (8) abgezogen werden. Das über die Leitung (9) abgezogene Gemisch kann direkt in den Spaltreaktor (15) eingesetzt werden. In diesem Falle würde die Leitung (9) direkt in die Leitung (13) einmünden und ein Teil des Gemisches wäre über die Leitung (14) und die Leitung (18) in den Veretherungsreaktor zurückzuführen. Vielfach besteht jedoch der Wunsch, die Bildung von reinen $C_4$-$C_7$-tert.-Alkenen mit der Entnahme bestimmter Ether mit 2- und/oder 3-wertigen Alkoholen zu verbinden. In einem solchen Fall, der in Fig. 1 gezeigt wird, mündet die Leitung (9) in eine Vakuumdestillationskolonne (10) in der als Kopfprodukt (11) einer oder mehrere Ether abgezogen werden, während über die Sumpfleitung (12) das zu spaltende Ethergemisch abgezogen und über (13) in (15) eingeleitet wird. Vorher wird auch hier über (14) ein Teil der Ether über (18) in die Veretherungsstufe zurückgeführt. Im Falle der Veretherung von i-Buten mit Propandiol-1,2 kann auf diese Weise über (11) 1-tert.-Butoxy-2-propanol als reine Substanz abgezogen werden, während ein Gemisch aus 2-tert.-Butoxy-1-propanol, 1,2-Di-tert.-butoxy-propan und etwa überschüssigem Propandiol-1,2 in die Etherspaltung in (15) geführt werden. Das Spaltprodukt, das (15) verläßt wird in die Destillationskolonne (16) eingeführt, in der über Kopf bei (17) das reine tertiäre Alken abgenommen wird, während der bei der Spaltung zurückbleibende 2-und/oder 3-wertige Alkohol sowie etwa nicht vollständig gespaltener Ether als Sumpfprodukt über die Leitung (18) in die Veretherung zurückgeführt wird. Ein solcher über (11) entnommener Monoether ist als wertvolles Lösungsmittel, als Lackrohstoff oder als Detergens verwertbar. Für den Fall einer solchen Produktentnahme über (11) muß der damit aus dem System gezogene 2-und/oder 3-wertige Alkohol über (2) im entsprechenden Maße nachgesetzt werden.

Beispiel 1

Als Spaltkontakt wurde ein makroporöser, starksaurer Kationenaustauscher (Styrol-Divinylbenzol-Harz mit $SO_3H$-Gruppen, z.B. SPC-118 von Bayer AG) eingesetzt.

In einem Büchi-Rührkessel mit aufgesetzter Vigreuxkolonne, gekühltem Rückflußkühler und gekühltem Abscheider wurde an diesem Kontakt ein Gemisch aus Diethylenglykol-mono-tert.-butylether und Diethylenglykol-di-tert.-butylether gespalten.

Die Gasphase und die Flüssigphase wurden gaschromatographisch untersucht.

Die Reaktionsbedingungen waren:

T. 95°C;      p = 1 bar;      t = 0,3 h

Die Produktverteilung ist in der nachstehenden Tabelle aufgelistet.

| | | Ausgang | |
|---|---|---|---|
| Produkt | Einsatz | Flüssig-phase | Gas-phase |
| Sonstige KW [Gew.-%] | 0,3 | 0,4 | |
| i-Buten " | < 0,1 | < 0,1 | > 99,9 |
| TBA " | 2,2 | 0,1 | |
| DEG " | 21,9 | 89,3 | |
| DEG-ME " | 46,8 | 9,9 | |
| DEG-DE " | 28,8 | 0,3 | |

```
Umsatz DEG-ME          85,1 %
Umsatz DEG-DE        ) 99,3 %


DEG     = Diethylenglykol
DEG-ME = Diethylenglykol-mono-tert.-butylether
DEG-DE = Diethylenglykol-di-tert.-butylether.
```

Beispiel 2 (Vergleichsbeispiel, Spaltung von Methyl-tert.-butylether, MTBE)

In den Zwangsumlauf einer Druck-Destillationskolonne wurde ein Reaktor, der mit dem Kontakt aus Beispiel 1 gefüllt war, eingebunden.

Die Zudosierung des MTBE erfolgte am Sumpf.

Ein Teil der Kolonne wurde als Waschstrecke genutzt. Zur Entfernung des gebildeten Methanols wurde Wasser zudosiert und das Wasser-Methanolgemisch über einen Abscheider als Seitenstrom der Kolonne entnommen.

Nach 24 h konstanter Fahrweise wurden Analysen gezogen und gaschromatographisch untersucht.

Die Spaltbedingungen waren:

T = 101°C;        p = 3,5 bar;        LHSV = 5

Die Produktverteilung ist in der nachstehenden Tabelle aufgeführt.

| Produkt | | Einsatz | Spaltprodukt (i-Buten-Fraktion |
|---|---|---|---|
| i-Buten | [ % ] | < 0,1 | 99,4 % |
| DME | " | < 0,1 | 0,5 % |
| MeOH | " | < 0,1 | 0,1 % |
| MTBE | " | > 99,9 | < 0,1 % |

```
DME  = Dimethylether
MeOH = Methanol
MTBE = Methyl-tert.-butyl-ether.
```

Beispiel 3

Der Versuch wurde analog zu Beispiel 1 durchgeführt, als Ether wurde ein Gemisch aus Dipropylenglykol-mono-tert.-butyl-ether und Dipropylenglykol-di-tert.-butyl-ether eingesetzt.

Die Reaktionsbedingungen waren:

T = 95°C;        p = 1 bar;        t = 0,3 h

Die erhaltene Produktverteilung ist in der nachstehenden Tabelle aufgelistet.

| Produkt | Einsatz | Ausgang | |
| --- | --- | --- | --- |
| | | Flüssig-phase | Gas-phase |
| Sonstige KW [Gew.-%] | 0,1 | 0,2 | |
| i-Buten " | < 0,1 | < 0,1 | > 99,9 |
| TBA " | < 0,1 | < 0,1 | |
| DPG-ME " | 91,5 | 7,4 | |
| DPG-DE " | 7,4 | < 0,1 | |
| DPG " | 1,0 | 92,4 | |

Umsatz DPG-ME        94,5 %

Umsatz DPG-DE      > 99,9 %

TBA      = Tertiärbutanol

DPG      = Dipropylenglykol

DPG-ME = Dipropylenglykol-mono-tert.-butyl-ether

DPG-DE = Dipropylenglykol-di-tert.-butyl-ether.

Beispiel 4

Die Etherspaltung wurde in einem mantelbeheizten Rohrreaktor mit einem lichten Durchmesser von 20 mm in der flüssigen Phase durchgeführt.

Als Spaltprodukt wurde ein Gemisch aus Monopropylenglykol-mono-tert.-butyl-ether und Monopropylenglykol-di-tert.-butyl-ether eingesetzt.

Das Reaktionsprodukt wurde in einem gekühlten Abscheider kondensiert und gaschromatographisch untersucht.

Die Reaktionsbedingungen waren:

Spalttemperatur 100° C; p = 1 bar; LHSV = 1; 2; 4.

Die Ergebnisse sind in der nachstehenden Tabelle aufgelistet.

| Produkt [Gew.-%] | LHSV 1 | LHSV 2 | LHSV 4 |
|---|---|---|---|
| i-Butan " | 0,7 | 0,4 | 0,1 |
| i-Buten " | 99,3 | 99,6 | 99,9 |
| $C_8^=$ " | 8,1 | 4,0 | 0,6 |
| Umsatz MPG-ME [% v.E] | 89,5 | 87,5 | 83,2 |
| Umsatz MPG-DE " | 92,0 | 96,9 | 94,6 |

$C_8^=$: $C_8$-Olefine

MGP-ME = Monopropylenglykol-mono-tert.-butyl-ether

MGP-DE = Monopropylenglykol-di-tert.-butyl-ether.

Beispiel 5

Der Versuch wurde analog zu Beispiel 4 durchgeführt.
Als Spaltkatalysator wurde ein Sulfonsäuregruppen enthaltendes Polysiloxan eingesetzt.
Spalttemperatur: 140° C; p = 1 bar; LHSV = 4

| Produkt [Gew.-%] | LHSV=4 |
|---|---|
| i-Butan " | 0,02 |
| i-Buten " | 99,98 |
| $C_8$-Olefine " | ‹0,1 |
| Umsatz MPG-ME | 92,7% |
| Umsatz MPG-DE | 95,6% |

**Patentansprüche**

1. Verfahren zur Herstellung von reinen $C_4$-$C_7$-tert.-Alkenen durch Spaltung ihrer Ether an sauren Katalysatoren bei erhöhter Temperatur, dadurch gekennzeichnet, daß man die $C_4$-$C_7$-tert.-Ether von 2- und/oder 3-wertigen Alkoholen einsetzt und bei 60-250° C spaltet.

2. Verfahren nach Anspruch 1, dadurch gekennzeichnet, daß man $C_4$-$C_7$-Ether von 2-wertigen Alkoholen

7

oder deren Oligoethern einsetzt.

3. Verfahren nach Anspruch 2, dadurch gekennzeichnet, daß man $C_4$-$C_7$-tert.-Ether von Ethylenglykol, Propandiol-1,2 oder deren Diethern einsetzt.

4. Verfahren nach Anspruch 1, dadurch gekennzeichnet, daß man Ether von i-Buten, 2-Methyl-buten-1 oder 2-Methyl-buten-2 einsetzt.

5. Verfahren nach Anspruch 1, dadurch gekennzeichnet, daß man als Spaltkatalysator ein Sulfonsäuregruppen enthaltendes Styrol-Divinylbenzol-Polymer in der $H^+$-Form bei 60-130° C einsetzt.

6. Verfahren nach Anspruch 1, dadurch gekennzeichnet, daß man als Spaltkatalysator ein Sulfonsäuregruppen enthaltendes Polyalkylsiloxan in der $H^+$-Form bei 60-160° C einsetzt.

7. Verfahren nach Anspruch 1, dadurch gekennzeichnet, daß man einen Ether einsetzt, der durch Umsetzung eines ein $C_4$-$C_7$-tert.-Alken enthaltenden Kohlenwasserstoffstroms mit einem 2- und/oder 3-wertigen Alkohol an einem Sulfonsäuregruppen enthaltenden Styrol-Divinylbenzol-Polymer in der $H^+$-Form in Gegenwart von 10 bis 40 Gew.-% des zu spaltenden Ethers bei 20-90° C erhalten wird, wobei die Spalttemperatur 20-120° C über der Veretherungstemperatur liegt.

8. Verfahren nach Anspruch 7, dadurch gekennzeichnet, daß man den bei der Spaltung zurückgewonnenen 2- und/oder 3-wertigen Alkohol gemeinsam mit nicht gespaltenem Ether in die Veretherung mit dem $C_4$-$C_7$-tert.-Alken zurückführt.

9. Verfahren nach Anspruch 8, dadurch gekennzeichnet, daß man Propandiol-1,2-mono- und/oder -di-tert.-$C_4$-$C_7$-alkylether spaltet und bei der Spaltung erhaltenes Propandiol-1,2, gemeinsam mit nicht gespaltenem Mono- und/oder Diether oder mit nicht vollständig gespaltenem Diether in die Umsetzung zur Veretherung zurückführt.

10. Verfahren nach Anspruch 7, dadurch gekennzeichnet, daß in der Umsetzung zur Veretherung 0,4-4 Mol $C_4$-$C_7$-tert.-Alken pro Äquivalent OH-Gruppe eingesetzt werden.

## Claims

1. Process for producing pure $C_4$-$C_7$-tert.-alkenes by cleavage of their ethers over acid catalysts at elevated temperature, characterised in that the $C_4$-$C_7$-tert.-ethers of dihydric and/or trihydric alcohols are used and cleaved at from 60-250°C.

2. Process according to Claim 1, characterised in that $C_4$-$C_7$-ethers of dihydric alcohols or oligoethers thereof are used.

3. Process according to Claim 2, characterised in that $C_4$-$C_7$-tert.-ethers of ethylene glycol, propane-1,2-diol or diethers thereof are used.

4. Process according to Claim 1, characterised in that ethers of iso-butene, 2-methyl-but-1-ene or 2-methyl-but-2-ene are used.

5. Process according to Claim 1, characterised in that, as cleavage catalyst, a styrene-divinylbenzene polymer containing sulphonic acid groups in the $H^+$-form is used at from 60-130°C.

6. Process according to Claim 1, characterised in that, as cleavage catalyst, a polyalkylsiloxane containing sulphonic acid groups in the $H^+$-form is used at from 60-160°C.

7. Process according to Claim 1, characterised in that the ether used is obtained by reaction at from 20-90°C of a hydrocarbon stream containing a $C_4$-$C_7$-tert.-alkene with a dihydric and/or trihydric alcohol over a styrene-divinylbenzene polymer containing sulphonic acid groups in the H+-form in the presence of from 10 to 40 % by weight of the ether to be cleaved, the cleavage temperature being 20-120°C above the etherification temperature.

8. Process according to Claim 7, characterised in that the dihydric and/or trihydric alcohol recovered from the cleavage, together with uncleaved ether, are recirculated back into the etherification with the $C_4$-$C_7$-tert.-alkene.

9. Process according to Claim 8, characterised in that propane-1,2-diol mono- and/or di-tert.-$C_4$-$C_7$-alkyl ethers are cleaved and the propane-1,2-diol obtained in the cleavage, together with uncleaved mono- and/or diether or with incompletely cleaved diether, are recirculated back into the etherification reaction.

10. Process according to Claim 7, characterised in that 0.4-4 mol of $C_4$-$C_7$-tert.-alkene per equivalent of OH group are used in the etherification reaction.


**Revendications**

1. Procédé de préparation de tert.-alcènes purs en $C_4$-$C_7$ par dissociation de leurs éthers sur des catalyseurs acides à température élevée, caractérisé en ce que l'on utilise des tert.-éthers en $C_4$-$C_7$ d'alcools bi- et/ou trivalents et qu'on les dissocie entre 60 et 250°C.

2. Procédé selon la revendication 1, caractérisé en ce que l'on utilise des éthers en $C_4$-$C_7$ d'alcools bivalents ou leurs oligoéthers.

3. Procédé selon la revendication 2, caractérisé en ce que l'on utilise des tert.-éthers en $C_4$-$C_7$ de l'éthylè-neglycol, du propanediol-1,2 ou leurs diéthers.

4. Procédé selon la revendication 1, caractérisé en ce que l'on utilise des éthers du i-butène, de 2-méthylbutène-1 ou de 2-méthylbuténe-2.

5. Procédé selon la revendication 1, caractérisé en ce que l'on utilise comme catalyseur de dissociation un polymère de styrène-divinylbenzène contenant des groupes d'acide sulfonique sous forme $H^+$ entre 60 et 130°C.

6. Procédé selon la revendication 1, caractérisé en ce que l'on utilise comme catalyseur de dissociation un polyalkylsiloxane contenant des groupes d'acide sulfonique sous forme $H^+$ entre 60 et 160°C.

7. Procédé selon la revendication 1, caractérisé en ce que l'on utilise un éther qui est obtenu en faisant réagir un courant d'hydrocarbures contenant un tert.-alcène en $C_4$-$C_7$ avec un alcool bi- et/ou trivalent sur un polymère de styrène-divinylbenzène contenant des groupes d'acide sulfonique sous forme $H^+$ en présence de 10 à 40% en poids de l'éther à dissocier, à une température comprise entre 20 et 90°C, dans lequel la température de dissociation est supérieure de 20 à 120 a la température d'éthérification.

8. Procédé selon la revendication 7, caractérisé en ce que l'alcool bi- et/ou trivalent provenant de la dissociation est renvoyé avec l'éther non dissocié à l'éthérification avec le tert.-alcène en $C_4$-$C_7$.

9. Procédé selon la revendication 8, caractérisé en ce que l'on dissocie le mono- et/ou le di-tert.-alkyléther en $C_4$-$C_7$ du propanediol-1,2 et que l'on recycle le propanediol-1,2 provenant de la dissociation avec le mono- et/ou diéther non dissocié ou avec le diéther non complètement dissocié à la réaction d'éthérification.

10. Procédé selon la revendication 7, caractérisé en ce que l'on utilise pour la réaction d'éthérification de 0,4 à 4 moles de tert.-alcènes en $C_4$-$C_7$ par équivalent de groupe OH.

FIG.1

EP 0 407 840 B1